# FASCICULE DE BREVET EUROPEEN

(11) **EP 3 157 368 B1**
(45) Date de publication et mention de la délivrance du brevet: **27.12.2017**
(21) Numéro de dépôt: 15734464.9
(22) Date de dépôt: 15.06.2015
(51) Int. Cl.: A42B 3/18, A61F 9/02

(54) **CASQUE ÉQUIPÉ DE LUNETTES DE PROTECTION, NOTAMMENT POUR LA PRATIQUE DU SKI OU DE LA MOTO**
MIT BRILLE AUSGESTATTETER HELM, INSBESONDERE ZUM SKI- ODER MOTORRADFAHREN
HELMET EQUIPPED WITH GOGGLES, IN PARTICULAR FOR SKIING OR MOTORCYCLING

(30) Priorité: 19.06.2014 FR 1455674
(43) Date de publication de la demande: 26.04.2017
(73) Titulaire: 7B+, 74000 Annecy (FR)
(72) Inventeur: LAPERRIERE, Christophe, F-74000 Annecy (FR)
(74) Mandataire: Jeannet, Olivier
(86) Numéro de dépôt international: PCT/IB2015/054517
(87) Numéro de publication internationale: WO 2015/193794

(56) Documents cités:
- EP-A2- 0 471 264
- GB-A- 2 464 749
- US-A- 3 781 915
- US-A- 5 845 341

## Description

La présente invention concerne un casque équipé de lunettes de protection, notamment pour la pratique du ski ou de la moto.

Le terme de "lunettes" doit être compris de manière large, englobant ce qu'il est convenu d'appeler, dans le domaine du ski, un "masque".

Il est très classique que des lunettes de protection destinées à être utilisées avec un casque comprennent une bande élastique engagée autour de la paroi du casque. Cette paroi étant lisse et glissante, ce montage classique a pour inconvénient d'induire une forte tendance de la bande à glisser vers le sommet du casque, par rappel élastique de cette bande. Ce glissement oblige l'utilisateur à retirer son casque pour pouvoir remettre les lunettes en place.

Ce montage classique a également pour inconvénient de soumettre la bande élastique à une tension importante lorsque l'utilisateur tire sur les lunettes afin de les déplacer entre la position d'utilisation, dans laquelle sont placées devant les yeux de l'utilisateur, et la position de non utilisation, dans laquelle elles sont placées sur la partie frontale du casque.

En outre, l'utilisateur ne peut généralement pas régler la tension de cette bande élastique sans enlever le casque.

La présente invention a pour objectif de remédier simultanément à l'ensemble de ces inconvénients.

Les publications de demandes de brevet N° GB 2 464 749, US 5,845,341, US 3,781,915 et EP 0 471 264 décrivent divers casques existants, ne permettant pas d'atteindre cet objectif.

Dans le but d'atteindre ledit objectif, le casque selon l'invention comprend :
- deux cordons non étirables élastiquement, reliés directement ou indirectement aux lunettes, et dimensionnés de manière à s'étendre jusqu'à la zone arrière du casque, en étant situés essentiellement à l'intérieur du casque, le long de la paroi de celui-ci ; un premier de ces cordons est relié à un côté latéral des lunettes et s'étend sur un premier côté latéral du casque, et le deuxième cordon est relié à l'autre côté latéral des lunettes et s'étend sur le deuxième côté latéral du casque ; et
- un mécanisme de traction simultanée sur les deux cordons, pour la mise en tension de ces cordons lorsque les lunettes sont placées dans une position d'utilisation, sur les yeux de l'utilisateur, et de relâchement simultané de ces deux cordons, afin de permettre de déplacer les lunettes vers une position de non utilisation, dans laquelle elles sont placées sur la partie frontale du casque, ce mécanisme incluant des moyens de maintien permettant de le maintenir dans au moins une position stable de traction desdits cordons.

L'invention consiste ainsi :
- à ne pas utiliser une bande élastique s'étendant sur la majeure partie de la périphérie du casque ni des portions de bande élastique s'étendant sur cette même majeure partie, mais des cordons non étirables élastiquement, et
- à opérer des tractions ou des relâchements simultanés sur ces cordons de manière à maintenir les lunettes en position d'utilisation ou à rendre possible le déplacement de ces lunettes vers la position de non utilisation.

Il sera compris que le terme "cordon" fait référence à des éléments filiformes, ayant une section transversale non aplatie, notamment circulaire, de faible largeur.

Le passage desdits cordons à l'intérieur du casque permet d'éliminer tout risque de glissement des lunettes le long de la face extérieure du casque. La faible largeur et la relativement faible section des cordons utilisés selon la présente invention, de même que la non élasticité de ces cordons, permet de réduire les frottements entre ces cordons et le casque, et donc d'assurer le parfait coulissement de ces cordons le long de la paroi du casque.

Ledit mécanisme permet de régler précisément la tension exercée sur les lunettes dans ladite position d'utilisation, avec une seule main et sans enlever le casque ; ce mécanisme permet également de relâcher suffisamment cette tension pour permettre d'amener les lunettes dans ladite position de non utilisation.

L'invention fournit de cette manière un casque équipé de lunettes permettant de parfaitement remédier aux inconvénients précités des casques homologues existants.

De préférence, les deux cordons sont reliés indirectement aux lunettes, le casque comprenant deux portions de bande élastique entre ces cordons et ces lunettes, chaque portions de bande élastique étant dimensionnée de manière à s'étendre depuis l'un des bords latéraux des lunettes de protection jusqu'à une zone comprise entre la partie avant de la zone temporale du casque et la partie arrière de cette même zone temporale.

L'invention utilise ainsi des portions de bande élastique assez courtes reliées aux cordons, qui permettent de maintenir confortablement les lunettes en place dans ladite position de non utilisation grâce à l'élasticité qu"elles ont.

De préférence, le casque comprend des conduits délimités, d'une part, par la paroi du casque, et, d'autre part, par des gouttières aménagées dans une doublure interne en matériau cellulaire que comprend le casque, lesdits cordons passant dans ces conduits et coulissant dans ceux-ci.

Ces conduits assurent un parfait guidage et un parfait coulissement des cordons.

De préférence, les portions de bande élastique sont reliées aux cordons par des attaches à actionnement rapide.

Ces attaches permettent un interchangement facile des lunettes. Chaque attache peut être du type comprenant une partie mâle venant en prise avec une partie femelle par encliquetage. Elle peut également être du type de celles que l'on trouve sur des vêtements imperméables, formées par deux pièces identiques incluant une fente oblique et des portions venant en prise les unes avec les autres par interpénétration, chaque pièce étant apte à être engagée dans la fente de l'autre pièce lorsque les deux pièces forment entre elles un angle lointain de 180° et étant apte à venir en prise avec l'autre pièce lorsque les deux pièces sont amenées l'une contre l'autre.

Pour opérer la traction sur les cordons, ledit mécanisme peut comprendre une roue d'enroulement des deux cordons, ou peut comprendre un pignon sur deux côtés duquel sont engagées des crémaillères solidaires des extrémités des cordons. Ce mécanisme peut également comprendre deux tambours crantés en prise l'un avec l'autre de telle sorte que ces tambours soient contrarotatifs, l'un des tambours étant en prise avec une crémaillère solidaire de l'extrémité de l'un des cordons et l'autre tambour étant en prise avec une crémaillère solidaire de l'extrémité de l'autre cordon. Ce mécanisme peut aussi comprendre deux tiges filetées à pas inversés, reliées l'une à l'autre et engagées dans des trous taraudés correspondants de blocs reliés aux extrémités des cordons. Pour son actionnement, le mécanisme peut comprendre une molette solidaire en rotation de ladite roue d'enroulement ou dudit pignon ; ledit tambour cranté peut lui-même former un organe d'actionnement ; lesdites tiges filetées à pas inversés peuvent être reliées axialement à un tambour cranté formant une molette l'actionnement de ces tiges filetées en rotation.

Lesdits moyens de maintien peuvent être de toute forme connue pour obtenir diverses positions angulaires stables d'une pièce pivotante par rapport à une pièce de base, notamment sous la forme de nervures formées sur l'une de ces pièces et de cannelures formées dans l'autre de ces pièces, chaque nervure étant apte à passer d'une cannelure à une autre par légère déformation élastique de l'une et/ou l'autre de ces pièces, et étant apte à être normalement maintenue dans la cannelure dans laquelle elle se trouve lorsque la ou lesdites pièces ne sont pas déformées élastiquement. Selon une autre possibilité, une première desdites pièces présente une section transversale polygonale, par exemple hexagonale, et est reçue dans un conduit ajusté, de section polygonale correspondante, formé par la deuxième pièce : le positionnement des angles sortants de ladite première pièce dans les angles rentrants formés par ladite deuxième pièce définit une position angulaire stable de la première pièce dans la deuxième pièce, et chaque angle sortant est apte à passer d'un angle rentrant à l'autre par déformation élastique de l'une et/ou l'autre de ces pièces.

Selon une forme de réalisation de l'invention,
- chaque portion de bande élastique est dimensionnée de manière à s'étendre depuis un bord latéral des lunettes de protection jusqu'à la partie antérieure de chaque zone temporale du casque, et
- chaque cordon débouche au niveau de cette partie antérieure.

Selon une autre forme de réalisation de l'invention,
- chaque portion de bande élastique est dimensionnée de manière à s'étendre depuis un bord latéral des lunettes de protection jusqu'à la partie centrale ou la partie arrière de la zone temporale correspondante de la paroi du casque, sur l'extérieur de cette paroi ;
- le cordon correspondant débouche au niveau de cette zone temporale.

De préférence, dans ce cas :
- le casque comprend une zone renfoncée au niveau de chaque zone temporale, dans laquelle débouche le cordon ;
- chaque portion de bande élastique est reliée au cordon correspondant par une attache à actionnement rapide, et
- ladite zone renfoncée est fermée par un flasque monté de façon amovible sur le casque, délimitant, sur le côté antérieur du casque, une fente de passage de ladite portion de bande élastique.

Ce flasque permet de protéger ladite attache à actionnement rapide.

Le flasque a de préférence une forme telle qu'il vient dans la continuité de la paroi du casque et qu'en position de montage, il s'inscrive dans la courbure de cette paroi. Il ne fait donc pas particulièrement saillie par rapport à cette paroi dans sa position de montage.

L'invention sera bien comprise, et d'autres caractéristiques et avantages de celle-ci apparaîtront, en référence au dessin schématique annexé, lequel représente, à titre d'exemples non limitatifs, deux formes de réalisation possibles du casque concerné.
La figure 1 en est une vue de côté, selon une première forme de réalisation, avec des lunettes de protection dont il est équipé ;
la figure 2 en est une vue de côté, avec plusieurs coupes partielles, à savoir une coupe selon un plan antéropostérieur médian et une coupe selon un plan transverse ; les lunettes de protection sont, sur cette figure 2, dans une position de non utilisation ;
la figure 3 en est une vue de face, avec retrait partiel d'une partie d'une doublure interne en matériau cellulaire que comprend le casque ;
la figure 4 en est une vue de dessus, avec une coupe partielle selon l'axe d'un conduit s'étendant le long de la paroi du casque ;
les figures 5A à 8 sont des vues schématiques, sous différents angles, de quatre types possibles d'un mécanisme de tension/relâchement de cordons que comprend le casque ;
la figure 9A est une vue de côté des lunettes de protection, dudit mécanisme et desdits cordons ; des portions de bande élastique reliées auxdites lunettes sont équipées d'un premier type de pièces d'assemblage à actionnement rapide ;
la figure 9B est une vue en perspective de ce premier type de pièces d'assemblage, en position de non assemblage ;
la figure 9C est une vue de dessus de ce premier type de pièces d'assemblage, en position de non assemblage ;
la figure 10A est une vue similaire à la figure 9A mais avec lesdites portions de bande élastique équipées d'un deuxième type de pièces d'assemblage à actionnement rapide ;
la figure 10B est une vue similaire à la figure 9B de ce deuxième type de pièces d'assemblage ;
la figure 11 est une vue similaire à la figure 2, avec les lunettes de protection en position d'utilisation, et
la figure 12 est une vue similaire à la figure 11 d'un casque selon une deuxième forme de réalisation.

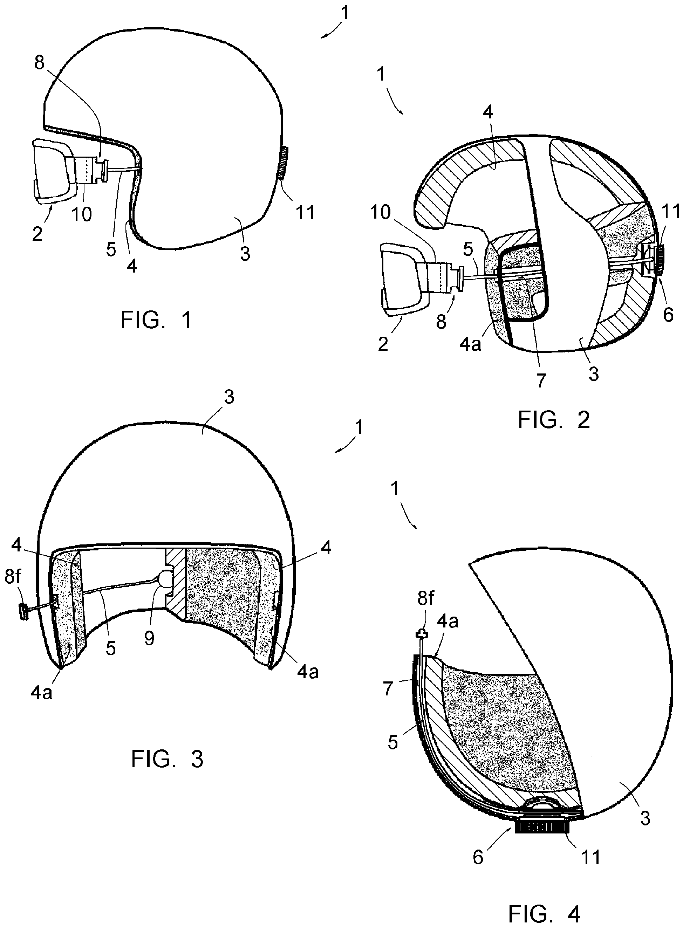

Les figures 1 à et 11 représentent un casque 1 équipé de lunettes de protection 2, notamment pour la pratique du ski ou de la moto.

Le casque 1 comprend une coque 3 et une doublure interne 4 en matériau cellulaire, d'absorption de chocs, ainsi qu'une sangle jugulaire de type connu (non représentée). Il comprend également deux cordons latéraux 5 et, sur son côté arrière, un mécanisme 6 auquel les cordons 5 sont reliés, permettant de réaliser une tension simultané des cordons 5 ou un relâchement simultané de ces cordons.

La coque 3 et la doublure interne 4 sont échancrées sur le côté antérieur du casque 1, de sorte que la doublure 4 présente deux faces de tranche 4a, latérales et antérieures.

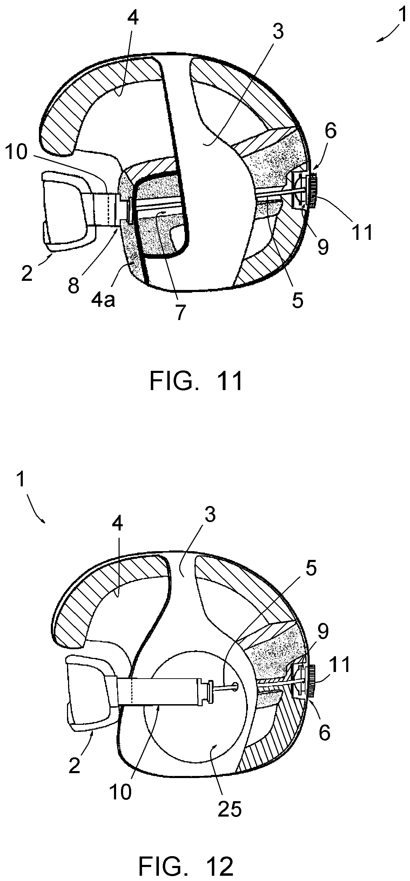

Cette coque 3 et cette doublure 4 délimitent entre elles deux conduits 7, un sur chaque côté latéral du casque 1, qui s'étendent entre un logement arrière de réception d'une partie du mécanisme 6 et lesdites faces 4a. Comme le montrent les figures 2 et 11, ces conduits 7 s'étendent sensiblement au niveau de la zone équatoriale du casque 1.

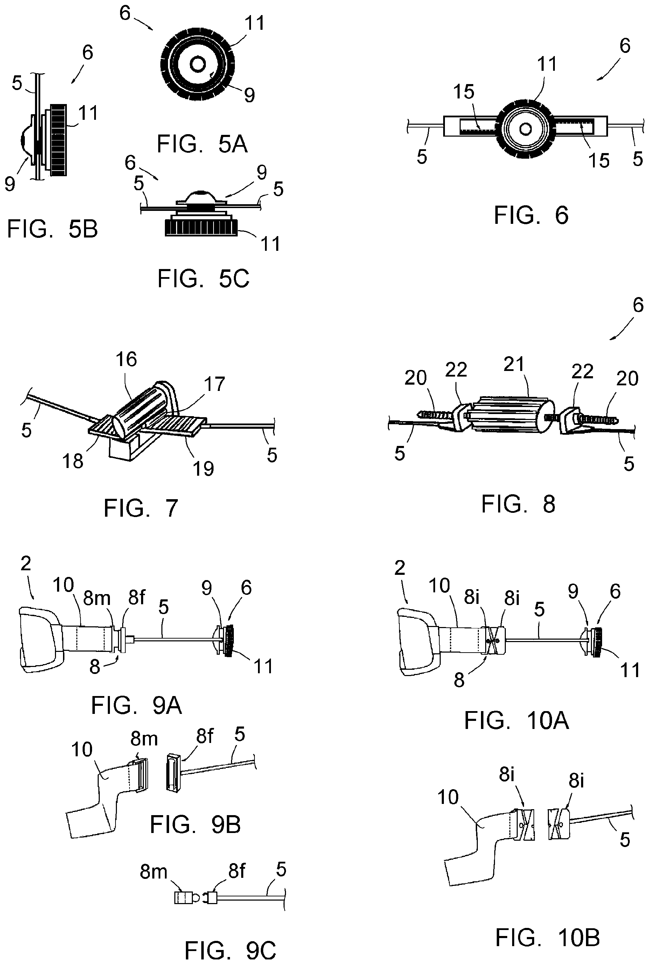

Chaque cordon 5 est filiforme, c'est-à-dire a une section transversale non aplatie, notamment circulaire, de quelques millimètres de largeur, par exemple de 2 à 4 mm de diamètre, et est non étirable longitudinalement. Il est engagé et est apte à coulisser dans l'un des conduits 7. Sur le côté antérieur du casque 1, l'extrémité de chaque cordon 5 est reliée à une pièce femelle 8f que comprend une attache 8 à actionnement rapide, qui sera décrite plus loin en référence aux figures 9A à 9C ; du côté du mécanisme 6, l'extrémité de chaque cordon 5 est reliée à une roue d'enroulement 9, décrite plus loin en référence aux figures 5A à 5C.

La paire de lunettes 2 présente une monture et des verres, ou un écran de protection, qui sont de type classique. Sur chaque côté latéral de la monture, la paire de lunettes 2 comprend une portion de bande 10 élastique, qui présente une longueur réduite, par exemple de 20 à 40 mm. Les deux portions de bande 10 sont réalisée avec le même type de matériau que celui d'une bande de lunettes homologues classique, étirable élastiquement dans la direction longitudinale. A leurs extrémités libres, chacune de ces deux portions de bande 10 comprend la partie mâle 8m de l'attache 8.

Les figures 5A à 5C représentent le mécanisme 6 respectivement selon l'axe de pivotement de ladite roue d'enroulement 9, de côté et de dessus. La roue d'enroulement 9 est reliée par un axe à une molette d'actionnement 11 destinée à être située sur l'extérieur de la coque 3. Cet axe présente une section transversale hexagonale et est reçu au travers d'un trou ajusté de section hexagonale correspondante, aménagé dans la coque 3 ; le positionnement des angles sortants de l'axe dans les angles rentrants formés par ce trou définit une position angulaire stable de l'axe dans le trou, et chaque angle sortant est apte à passer d'un angle rentrant à l'autre par déformation élastique de l'axe. L'ensemble molette 11 / roue 9 peut ainsi être déplacé entre une pluralité de positions angulaires données, en passant d'une position à une autre par franchissement de points durs, chaque position angulaire étant stable.

La molette 11 est apte à être actionnée par l'utilisateur lorsque le casque 1 est porté ; dans un premier sens de rotation, elle réalise l'enroulement des cordons 5 autour de la roue d'enroulement 9, et réalise donc une traction simultanée sur ces deux cordons, tandis que dans le deuxième sens de rotation, elle réalise le déroulement des cordons 5 vis-à-vis de la roue d'enroulement 9, et donc un relâchement simultané de ces deux cordons.

Comme cela se comprend en comparaison des figures 2 et 11, dans la position de relâchement des cordons 5, les lunettes 2 sont déplaçables vers une position antérieure et avancée par rapport au casque 1 (cf. figure 2), permettant que les lunettes 2 soient amenées dans une position de non utilisation, dans laquelle elles peuvent être placées sur la portion frontale du casque 1, sans qu'il soit nécessaire d'opérer une traction excessive sur les portions de bande 10 ; dans la position de traction des cordons 5, les lunettes 2 sont amenées vers une position postérieure d'utilisation, dans laquelle les attaches 8 sont reçues contre les faces 4a de la doublure 4 (cf. figure 11). Cette réception constitue un arrêt du déplacement des lunettes 2 et définit un positionnement idéal de ces lunettes 2 ; en outre, elle prévient le risque d'une traction excessive sur les cordons 5.

Comme visible sur cette figure 12, dans cette position d'utilisation, chaque portion de bande 10 est dimensionnée de manière à s'étendre jusqu'à la portion antérieure de chaque zone temporale du casque 1.

En référence aux figures 9A à 9C, il apparaît que la pièce femelle 8f de l'attache 8 comprend un bloc de base relié au cordon 5 et une gouttière réceptrice. La pièce mâle comprend quant à elle une embase formant un passant pour sa liaison à la portion de bande 10 et un jonc dimensionné de manière à être apte à être reçu de façon ajustée dans ladite gouttière réceptrice et à être retenu dans celle-ci dans la direction transversale à ce jonc.

La figure 6 montre une autre forme de réalisation du mécanisme 6, vue axialement, dans laquelle ce mécanisme comprend un pignon sur deux côtés duquel sont engagées des crémaillères 15 solidaires des extrémités des cordons 5.

La figure 7 montre une autre forme de réalisation du mécanisme 6, vue en perspective, dans laquelle ce mécanisme comprend deux tambours crantés 16, 17 en prise l'un avec l'autre de telle sorte que ces tambours soient contrarotatifs, l'un des tambours 16 étant en prise avec une crémaillère 18 solidaire de l'extrémité de l'un des cordons 5 et l'autre tambour 17 étant en prise avec une crémaillère 19 solidaire de l'extrémité de l'autre cordon 5.

La figure 8 montre encore une autre forme de réalisation du mécanisme 6, vue en perspective, dans laquelle ce mécanisme comprend deux tiges filetées 20 à pas inversés, reliées coaxialement l'une à l'autre à un tambour cranté 21 et engagées dans des trous taraudés correspondants de blocs 22 reliés aux extrémités des cordons 5.

Pour son actionnement, le mécanisme selon la figure 6 comprend une molette 11 identique à celle du mécanisme selon les figures 5A à 5C ; le tambour cranté 16 du mécanisme selon la figure 7, s'il fait saillie de la paroi de la coque 3, peut lui-même former un organe d'actionnement du mécanisme, de même que le tambour cranté 21 du mécanisme selon la figure 8.

Les figures 10A et 10B montrent une autre forme de réalisation de l'attache 8 ; dans ce cas, l'attache 8 est du type de celles que l'on trouve sur des vêtements imperméables, formées par deux pièces identiques 8i incluant une fente oblique et, de part et d'autre de cette fente, des portions venant en prise les unes avec les autres par interpénétration. Chaque pièce 8i est apte à être engagée dans la fente de l'autre pièce lorsque les deux pièces forment entre elles un angle lointain de 180° et est apte à venir en prise avec l'autre pièce lorsque les deux pièces sont amenées l'une contre l'autre.

La figure 12 montre une variante de réalisation du casque 1 et des lunettes 2, dans laquelle :
- le casque 1 forme une zone renfoncée 25 en creux, de forme circulaire, au niveau de chacune de ses zones temporales, dans laquelle débouche le cordon 5 correspondant ;
- chaque portion de bande 10 est dimensionnée de manière à s'étendre sur l'extérieur de la paroi de la coque 3, jusqu'à la partie centrale de la zone temporale correspondante du casque 1, donc jusqu'à l'intérieur de la zone renfoncée 25, et
- la zone renfoncée 25 est fermée par un flasque (non représenté) qui est monté de façon amovible sur le casque 1, ce flasque délimitant, sur le côté antérieur du casque 1, une fente de passage de ladite portion de bande 10.

Ce flasque permet de protéger l'attache 8. Il a de préférence une forme telle qu'il vient dans la continuité de la paroi de la coque 3 et qu'en position de montage, il s'inscrive dans la courbure de cette paroi, de manière à ne pas faire particulièrement saillie par rapport à cette paroi dans sa position de montage.

Ainsi que cela apparaît de ce qui précède, l'invention fournit un casque 1 équipé de lunettes de protection 2, notamment pour la pratique du ski ou de la moto, qui présente des avantages déterminants, précités.

L'invention a été décrite ci-dessus en référence à des formes de réalisation fournies à titre d'exemple. Il va de soi qu'elle n'est pas limitée à ces formes de réalisation et qu'elle s'étend à toutes les autres formes de réalisation couvertes par les revendications annexées.

## Revendications

1. Casque (1) équipé de lunettes de protection (2), notamment pour la pratique du ski ou de la moto, **caractérisé en ce qu'**il comprend :
- deux cordons (5) non étirables élastiquement, reliés directement ou indirectement aux lunettes (2) et dimensionnés de manière à s'étendre jusqu'à la zone arrière du casque (1), en étant situés essentiellement à l'intérieur du casque (1), le long de la paroi de celui-ci ; un premier de ces cordons (5) est relié à un côté latéral des lunettes (2) et s'étend sur un premier côté latéral du casque (1), et le deuxième cordon (5) est relié à l'autre côté latéral des lunettes et s'étend sur le deuxième côté latéral du casque (1) ; et
- un mécanisme (6) de traction simultanée sur les deux cordons (5), pour la mise en tension de ces cordons (5) lorsque les lunettes (2) sont placées dans une position d'utilisation, sur les yeux de l'utilisateur, et de relâchement simultané de ces deux cordons (5), afin de permettre de déplacer les lunettes (2) vers une position de non utilisation, dans laquelle elles sont placées sur la partie frontale du casque (1), ce mécanisme (6) incluant des moyens de maintien permettant de le maintenir dans au moins une position stable de traction desdits cordons (5).

2. Casque (1) selon la revendication 1, **caractérisé en ce que** les deux cordons (5) sont reliés indirectement aux lunettes (2), et **en ce que** le casque (1) comprend deux portions (10) de bande élastique entre ces cordons (5) et ces lunettes (2), chaque portions (10) de bande élastique étant dimensionnée de manière à s'étendre depuis l'un des bords latéraux des lunettes (2) jusqu'à une zone comprise entre la partie avant de la zone temporale du casque (1) et la partie arrière de cette même zone temporale.

3. Casque (1) selon la revendication 1 ou la revendication 2, **caractérisé en ce qu'**il comprend des conduits (7) délimités, d'une part, par la paroi du casque (1), et, d'autre part, par des gouttières aménagées dans une doublure interne (4) en matériau cellulaire que comprend le casque (1), et **en ce que** lesdits cordons (5) passent dans ces conduits (7) et coulissent dans ceux-ci.

4. Casque (1) selon la revendication 2 ou la revendication 3, **caractérisé en ce que** les portions (10) de bande élastique sont reliées aux cordons (5) par des attaches (8) à actionnement rapide.

5. Casque (1) selon l'une des revendications 1 à 4, **caractérisé en ce que** :
- chaque portion (10) de bande élastique est dimensionnée de manière à s'étendre depuis un bord latéral des lunettes de protection (2) jusqu'au bord latéral antérieur formé par chaque zone temporale casque (1), et
- chaque cordon (5) débouche au niveau dudit bord latéral antérieur.

6. Casque (1) selon l'une des revendications 1 à 4, **caractérisé en ce que** :
- chaque portion (10) de bande élastique est dimensionnée de manière à s'étendre depuis un bord latéral des lunettes de protection (2) jusqu'à la portion antérieure de chaque zone temporale du casque (1), et **en ce que**
- chaque cordon (5) débouche au niveau de cette portion antérieure.

7. Casque (1) selon la revendication 6, **caractérisé :**
- **en ce qu'**il comprend une zone renfoncée (25) au niveau de chaque zone temporale, dans laquelle débouche le cordon (5) ;
- **en ce que** chaque portion (10) de bande élastique est reliée au cordon (5) correspondant par une attache (8) à actionnement rapide, et
- **en ce que** ladite zone renfoncée (25) est fermée par un flasque monté de façon amovible sur le casque (1), délimitant, sur le côté antérieur du casque (1), une fente de passage de ladite portion (10) de bande élastique.

8. Casque (1) selon la revendication 7, **caractérisé en ce que** le flasque a une forme telle qu'il vient dans la continuité de la paroi du casque (1) et qu'en position de montage, il s'inscrive dans la courbure de cette paroi.

## Patentansprüche

1. Mit Schutzbrille (2) ausgestatteter Helm (1), insbesondere zum Skifahren oder Motorradfahren, **dadurch gekennzeichnet, dass** er umfasst:
- zwei, nicht elastische dehnbare Schnüre (5), die direkt oder indirekt mit der Brille (2) verbunden sind und so dimensioniert sind, dass sie sich bis zum hinteren Bereich des Helms (1) erstrecken, der sich im Wesentlichen innerhalb des Helms befindet (1) entlang der Wand davon; eine erste dieser Schnüre (5) ist mit einer lateralen Seite der Brille (2) verbunden und erstreckt sich auf einer ersten lateralen Seite des Helms (1), und die zweite Schnur (5) ist mit der anderen lateralen Seite der Brille (2) verbunden und erstreckt sich auf der zweiten Seite des Helms (1); und
- einen Mechanismus (6) zum gleichzeitigen Ziehen an den beiden Schnüren (5) zum Spannen dieser Schnüre (5), wenn die Brille (2) in einer Gebrauchsstellung an den Augen des Benutzers angebracht ist und gleichzeitige Freigabe dieser beiden Schnüre (5), um zu ermöglichen, die Brille (2) in eine Nichtgebrauchsstellung zu bewegen, in der sie auf dem vorderen Teil des Helmes (1) angeordnet sind, wobei dieser Mechanismus (6) einschließlich Haltemittel, um es in mindestens einer stabilen Traktionsposition der Schnüre (5) zu halten.

2. Helm (1) nach Anspruch 1, **dadurch gekennzeichnet, dass** die zwei Schnüre (5) indirekt mit der Brille (2) verbunden sind, und dass der Helm (1) zwei Abschnitte (10) aus elastischem Band zwischen diesen Schnüre (5) aufweist und diese Brillen (2), wobei jeder Abschnitt (10) des elastischen Bandes so bemessen ist, dass er sich von einem der seitlichen Ränder der Brille (2) zu einem Bereich zwischen dem vorderen Bereich des Temporalzone des Helms (1) erstreckt. und der hintere Teil derselben Temporalzone.

3. Helm (1) nach Anspruch 1 oder Anspruch 2, **dadurch gekennzeichnet, dass** er Kanäle (7) aufweist, die einerseits durch die Wand des Helms (1) begrenzt sind und zweitens durch Rinnen, die in einer inneren Auskleidung (4) aus zellularem Material angeordnet sind, die den Helm (1) umfasst, und dass die Schnüre (5) durch diese Kanäle (7) hindurchgehen und darin gleiten.

4. Helm (1) nach Anspruch 2 oder Anspruch 3, **dadurch gekennzeichnet, dass** die Abschnitte (10) des elastischen Bandes mit den Schnüren (5) durch Befestigungselemente (8) auf schnelle Betätigung verbunden sind.

5. Helm (1) nach einem der Ansprüche 1 bis 4, **dadurch gekennzeichnet, dass**:
- jeder Abschnitt (10) des elastischen Bandes ist so bemessen, dass er sich von einer seitlichen Rand der Brille (2) zu der vorderen Seitenrand erstreckt, die von jeder Temporalzone des Helms (1) gebildet wird, und
- jeder Schnur (5) öffnet sich an dem vorderen Seitenrand.

6. Helm (1) nach einem der Ansprüche 1 bis 4, **dadurch gekennzeichnet, dass**:
- jeder Abschnitt (10) des elastischen Bandes ist so bemessen, dass er sich von einer seitlichen Rand der Brille (2) zum vorderen Abschnitt jeder Temporalzone des Helms (1) erstreckt, und
- jeder Schnur (5) öffnet sich an diesem vorherigen Abschnitt.

7. Helm (1) nach Anspruch 6, **gekennzeichnet durch**:
- daß sie in jeder Temporalzone einen ausgesparten Bereich (25) aufweist, in den sich der Schnur (5) öffnet;
- dass jeder Abschnitt (10) des elastischen Bandes mit der entsprechenden Schnur (5) **durch** einen Schnellverschluss (8) verbunden ist und
- dass der vertiefte Bereich (25) **durch** einen Flansch geschlossen ist, der entfernbar an dem Helm (1) angebracht ist und an der Vorderseite des Helms (1) einen Schlitz zum Durchführen des Abschnitts (10) des elastischen Bandes definiert.

8. Helm (1) nach Anspruch 7, **dadurch gekennzeichnet, dass** der Flansch eine solche Form aufweist, dass er in die Kontinuität der Wand des Helms (1) eingeht und in der Montageposition Teil der Krümmung dieser Wand sich versetzt.

## Claims

1. Helmet (1) equipped with goggles (2), especially for skiing or motorcycling, **characterized in that** it comprises:
- two elastic non-stretchable cords (5), linked directly or indirectly to the goggles (2) and sized in such a way as to extend to the rear area of the helmet (1), being located essentially inside the helmet (1), along the wall thereof; a first of these cords (5) is linked to a lateral side of the goggles (2) and extends over a first lateral side of the helmet (1), and the second cord (5) is linked to the other lateral side of the goggles and extends over the second lateral side of the helmet (1); and
- a mechanism (6) for simultaneous traction on the two cords (5), for tensioning of these cords (5) when the goggles (2) are placed in a position of use, on the eyes of the user, and for simultaneously releasing these two cords (5), in order to allow the goggles (2) to be moved to a non-use position, in which they are placed on the front part of the helmet (1), this mechanism (6) including holding means for maintaining it in at least one stable tensioning position of said cords (5).

2. Helmet (1) according to claim 1, **characterized in that** the two cords (5) are linked indirectly to the goggles (2), and **in that** the helmet (1) comprises two portions (10) of elastic band between these cords (5) and these goggles (2), each portion (10) of elastic band being so dimensioned as to extend from one of the lateral edges of the goggles (2) to an area extending between the front portion of the temporal zone of the helmet (1) and the rear part of this same temporal zone.

3. Helmet (1) according to claim 1 or claim 2, **characterized in that** it comprises ducts (7) delimited, on the one hand, by the wall of the helmet (1), and, on the other hand, by gutters arranged in an inner lining (4) of cellular material that comprises the helmet (1), and **in that** said cords (5) pass through these ducts (7) and slide therein.

4. Helmet (1) according to claim 2 or claim 3, **characterized in that** the portions (10) of elastic band are linked to the cords (5) by quick-actuation fasteners (8).

5. Helmet (1) according to one of claims 1 to 4, **characterized in that**:
- each portion (10) of elastic band is so dimensioned as to extend from a lateral edge of the goggles (2) to the front lateral edge formed by each temporal zone of the helmet (1), and
- each cord (5) extends from said front lateral edge.

6. Helmet (1) according to one of claims 1 to 4, **characterized in that**:
- each portion (10) of elastic band is sized so as to extend from a lateral edge of the goggles (2) to the front portion of each temporal zone of the helmet (1), and **in that**
- each cord (5) extends from this prior portion.

7. Helmet (1) according to claim 6, **characterized:**
- **in that** it comprises a recessed area (25) at each temporal zone, from which the cord (5) extends;
**in that** each portion (10) of elastic band is linked to the corresponding cord (5) by a quick-actuation fastener (8), and
- **in that** said recessed area (25) is closed by a cover detachably mounted on the helmet (1), delimiting, on the front side of the helmet (1), a slot for passage of said portion (10) of elastic band.

8. Helmet (1) according to claim 7, **characterized in that** the cover has such a shape that it comes in the continuity of the wall of the helmet (1) and **in that**, in the mounting position, it follows the extension of the curvature of this wall.
